# EUROPÄISCHE PATENTSCHRIFT

(11) **EP 2 730 298 B1**
(45) Veröffentlichungstag und Bekanntmachung des Hinweises auf die Patenterteilung: **18.05.2016**
(21) Anmeldenummer: 12192006.0
(22) Anmeldetag: 09.11.2012
(51) Int. Cl.: A61L 27/44, A61L 27/04, A61L 27/18, A61L 27/40, A61F 2/28

(54) **Knochenimplantat aus mindestens zwei unterschiedlichen, resorbierbaren und biodegradierbaren Materialien, die als Hybrid- oder Composite-Material kombinierbar sind**
Bone implant made of at least two different resorbable and biodegradable materials, which can be combined as a hybrid or composite material
Implant osseux constitué d'au moins deux matériaux différents résorbables et biodégradables, pouvant être combinés en un matériau hybride ou composite

(43) Veröffentlichungstag der Anmeldung: 14.05.2014
(73) Patentinhaber: Karl Leibinger Medizintechnik GmbH & Co. KG, 78570 Mühlheim/Donau (DE)
(72) Erfinder: Gabele, Lorenz, 78570 Mühlheim (DE); Reinauer, Frank, 78570 Mühlheim (DE); Wolfram, Tobias, 78570 Mühlheim (DE); Müller, Wolfgang, 78570 Mühlheim (DE)
(74) Vertreter: Winter, Brandl, Fürniss, Hübner, Röss, Kaiser, Polte - Partnerschaft mbB

(56) Entgegenhaltungen:
- EP-A1- 0 502 349
- EP-A2- 1 683 593
- WO-A1-2011/006354
- WO-A2-2009/116799
- WO-A2-2010/101901
- DE-A1-102006 047 663
- US-A- 4 550 448
- US-A1- 2008 249 638

## Beschreibung

### Gebiet der Erfindung

Die Erfindung betrifft ein Knochenimplantat mit den Merkmalen des Oberbegriffs des Anspruchs 1.

Aus dem Stand der Technik sind bereits resorbierbare, bio-degradierbare Materialien, wie z.B. PDLLA, PGA oder PCL bekannt. Sie haben jedoch deutlich geringere mechanische Festigkeitseigenschaften als Standardmetallwerkstoffe, wie z.B. Titan oder Implantatstahl.

Leider weisen bio-degradierbare keramische Implantatmaterialien in vielen Bereichen eine zu geringe Bruch- und Biege-Wechselfestigkeit auf und sind oftmals nur schwer oder auch nicht "chair-side" modellierbar.

Das Verwenden von Magnesium und Magnesiumlegierungen in dem Bereich von Implantaten, ist zwar grundsätzlich bekannt, insbesondere da sie hohe Festigkeiten ermöglichen, allerdings erfolgt die Resorption nur unter suboptimalen Bedingungen in puncto Kinetik, physiologischer Aspekte, Gasentwicklung, Abbaumechanismen und Abbauprodukte, bei Anwendung am Knochen eines Säugetiers und insbesondere dem Menschen. Die Resorption erfolgt häufig zu schnell.

Relevanter Stand der Technik ist aus der WO 2009/116799 A2, der US 2008/0249638 A1, der WO 2011/006354 A1, der WO 2010/101901 A2, der US 4,550,448 A, der EP 1 683 593 A2, der DE 10 2006 047663 A1 und der EP 0 502 349 A1 bekannt.

Es ist die Aufgabe der vorliegenden Erfindung, die aus dem Stand der Technik bekannten Nachteile zu vermeiden und ein optimal resorbierendes Knochenimplantat zur Verfügung zu stellen, das zu jedem Zeitpunkt der Knochenregeneration eine ausreichend hohe Festigkeit aufweist. Zusätzlich soll ein solches Knochenimplantat kostengünstig sein und eine möglichst lange Lagerungszeit ohne Verfall der gewünschten Eigenschaften ermöglichen.

Die Materialverträglichkeit mit einem menschlichen Knochen und dem den menschlichen Knochen umgebenden Gewebe soll gewährleistet sein.

### Offenbarung der Erfindung

Dies wird erfindungsgemäß durch das Kennzeichen des Anspruchs 1 gelöst.

Auf diese Weise werden mindestens zwei oder auch mehrere bio-degradierbare Materialien, evtl. kombiniert mit nicht-degradierbaren Inhalts- oder Aktivstoffen, mit unterschiedlichen Eigenschaften zur definierten Einstellung der unterschiedlichen chemischen, physikalischen, mechanischen und biologischen Eigenschaften des Gesamtsystems eingesetzt.

Das Implantat weist eine hohe Festigkeit auf wenn bspw. Magnesium oder Magnesiumlegierungen eingesetzt werden. Mit bisher bekanntem Magnesium oder Magnesiumlegierungen erfolgte der Abbau dann aber für die wichtigen klinischen Applikationen am Knochen zu schnell. Wenn das Knochenimplantat jedoch erfindungsgemäß aus Magnesium oder einer Magnesiumlegierung in Kombination mit einer Schutzstruktur, die zwar gegebenenfalls eine geringe Festigkeit, aber eine optimale Degradationskinetik sicherstellt, gefertigt wird, wird eine verbesserte Ausführung möglich. Bei der erfindungsgemäßen Kombination der zwei oder mehr sich umgebenden oder durchsetzenden Werkstoffe kann die Tragstruktur vor zu schnellem Abbau geschützt werden. Eine Einstellung der Abbaudynamik/Kinetik wird damit möglich. Eine Einstellung der physiologischen/ metabolischen Aktivität im Umfeld des Implantats kann zielgerichtet durchgeführt werden. Auch ist es möglich, eine bedarfsabhängige Einstellung chemischer Bedingungen, wie z.B. des pH-Werts, der Konzentration der Abbauprodukte, etc., durchzuführen. Eine Einstellung der mechanischen Eigenschaften des originären Implantats und der Implantateigenschaften während der Abbauphase wird möglich. Während des Implantatzustandes wird sichergestellt, dass eine hohe mechanische Festigkeit aufgrund der Struktur der höherfesten, vorzugsweise metallischen Komponente gegeben ist. Der Abbau der mechanischen Festigkeit wird durch den Aufbau von Knochenmaterial kompensiert. Die höherfeste Komponente resorbiert zwar, wohingegen die Hüllstruktur der niedrigfesten Komponente bspw. länger erhalten bleibt und die höherfeste Komponente vor einer ggfs. Zu schnellen Resorption schützt. Durch die Schutzummantelung des höherfesten Materials mittels der niedrigfesten Komponente, ergibt sich bei entsprechender Einstellung der Resorptionskinetik eine z.B. mechanische Schutzfunktion für das umliegende Gewebe. Eine sinnvolle Art von Gewebeschutz lässt sich realisieren.

Vorteilhafte Ausführungsformen sind in den Unteransprüchen beansprucht und werden nachfolgend näher erläutert.

So ist es von Vorteil, wenn die Metalllegierung ein bio-degradierbarer und resorbierbarer Werkstoff ist, da dann auch die Tragstruktur komplett vom Körper abbaubar ist.

Auch ist es vorteilhaft, wenn die Schutzstruktur aus nicht-metallischem Material besteht oder dieses aufweist (beinhaltet).

Wenn der Werkstoff zumindest ein Element oder mehrere Elemente der Gruppe bestehend aus Magnesium, Eisen, Zink, Strontium, Fluor, Mangan und Calcium sowie deren mögliche Ionen aufweist, so kann auf besonders geeignete Werkstoffe und Werkstofflegierungen zurückgegriffen werden.

Zweckmäßig ist es, wenn die Schutzstruktur aus dem von der Tragstruktur unterschiedlichen Werkstoff besteht. Die unterschiedlichen Eigenschaften lassen sich dann bedarfsgerecht miteinander kombinieren.

Ein vorteilhaftes Ausführungsbeispiel ist auch dadurch gekennzeichnet, dass die Schutzstruktur aus Kunststoff besteht, und vorzugsweise Polylactid-Verbindungen Als vorteilhaft hat es sich auch herausgestellt, wenn die Schutzstruktur Polymilchsäure, wie PLA, und/oder Polyglykolsäure (PEA) und/oder Poly-Caprolacton (PCL), wie Poly-ε-Caprolacton-co-Lactid, PDLLA-TCP, PDLLA-Kalziumcarbonat und/oder PDA aufweist.

Um ein besonders vorteilhaftes Ausführungsbeispiel zu erreichen ist es von Vorteil, wenn die Tragstruktur und die Schutzstruktur nach Art eines Material-Composit-Bauteils oder eines Hybridbauteils voneinander durchdrungen oder miteinander vermischt sind, etwa nach Art eines Einkornbetons. Das Material-Composit hat in seinem gesamten 3D-Volumen gleichbleibende Abbaucharakteristika, so dass es das Knochenimplantat unempfindlich gegen oberflächliche mechanische Beeinträchtigung, z.B. bei der Ankonturierung, Verbiegung, Trennung und/oder mechanischen Bearbeitung macht. Die Nachteile, wie sie etwa beim sog. "coating" von Implantaten ohne Material-Composit auftreten, können dann vermieden werden. Häufig wird nämlich bei der mechanischen Bearbeitung des Implantats z.B. bei der Ankonturierung oder beim Trennen das Coating, also die Beschichtung, verletzt, d.h. der lokal auftretende "korosive" Angriff führt dann zu einer unerwünschten Abbaukinetik des Implantats an dieser Stelle, was bei der porösen, erfindungsgemäßen Ausgestaltung vermieden wird. Auch wird vermieden, dass beim Biegen die Schutzschicht verletzt, d.h. das darunter liegende Magnesiumimplantat freigesetzt und der Korrosion freigegeben wird, ggf. sogar an evtl. hochbelasteten Querschnitten.

Es ist auch von Vorteil, wenn die Tragstruktur eine Form aneinandergesetzter Tragpartikel, wie Körner, und/oder Kugeln, und/oder Fachwerkelemente, wie Stäbe, und/oder Hexagonalelemente, und/oder Dreiecke, und/oder krähenfußartiger Elemente, und/oder Waben, und/oder Fasern aufweist. Besonders belastbare und vielseitige Knochenimplantate lassen sich dann generieren.

Wenn die Tragstruktur die Geometrie eines Metallschaums aufweist, so lässt sich das Herstellen vereinfachen. Auch ist es von Vorteil, wenn die Tragpartikel so aneinander grenzen, dass zwischen ihnen Hohlräume befindlich sind. Das Einbringen der Schutzstruktur wird dadurch erleichtert.

Wenn zumindest ein Hohlraum oder vorzugsweise eine Vielzahl von Hohlräumen, etwa alle Hohlräume, mit dem Material der Schutzstruktur zumindest teilweise, vorzugsweise vollständig befüllt sind, so kann ein kompaktes Knochenimplantat mit besonders hoher Festigkeit erreicht werden, so dass das Knochenimplantat entsprechend der erfindungsgemäß eingestellten Abbaukinetik gegen Korrosion geschützt bleibt.

Besonders zweckmäßig ist es, wenn einige Hohlräume vollständig mit Material der Schutzstruktur ausgefüllt sind und einige Hohlräume nur teilweise mit Material der Schutzstruktur ausgefüllt sind, und vorzugsweise das Verhältnis von vollständig ausgefüllten Hohlräumen zu teilweise ausgefüllten Hohlräumen 20:1 bis 10:1, weiter vorzugsweise das Verhältnis ungefähr 15:1 ist.

Als besonders zweckmäßig hat es sich in Versuchen herausgestellt, wenn die Tragstruktur mit der Schutzstruktur verschränkt und/oder in Sandwich-Bauart miteinander kombiniert ist.

Damit die einzelnen Eigenschaften sinnvoll kombiniert werden können, ist es von Vorteil, wenn die Tragstruktur eine höhere Festigkeit als die Schutzstruktur aufweist.

Von Vorteil ist es auch, wenn das Knochenimplantat die Form einer Platte, eines Cranialknochenimplantats, eines Nagels, eines Gitters, eines Gewebes oder einer Schraube aufweist. Auch eine Nietform kann gewählt sein.

Vorteilhaft ist es ferner, wenn die Tragstruktur als Sinterstruktur ausgebildet ist. Es ist möglich, dass die einzelnen Composit-Bestandteile, wie Magnesiumkügelchen, an ihren Berührungsstellen miteinander in Herstellung einer Sinterstruktur "verschweißt" sind. Der freie Raum innerhalb des Composits ist z.B. mit PDLLA gefüllt und verhindert den zu schnellen korrosiven Angriff der Körperflüssigkeiten auf der Magnesium-Tragstruktur. Die Kügelchen können auch durch Regelgeometrien, die z.B. Honeycomb-Layers räumliche Dreiecke, nach Art einer Panzersperrengeometrie oder Fachwerkstrukturen ersetzt sein. Vorteilhaft sind weiterhin Strukturen, bei denen die Tragstruktur z.B. in Form der oben genannten Regelgeometrien in einer Sinterstruktur hergestellt sind.

Die Layer-Schichten können aus Metall und PDLLA in Sandwich-Bauweise kombiniert werden. Auch ist der Metallschaum mit PDLLA ausfüllbar.

Wenn die Schutzstruktur in Bereichen größerer mechanischer und/oder chemischer Belastung ungleich dicker, als in dazu benachbarten Bereichen auf der Tragstruktur vorhanden ist, so lässt sich auch bei hochbelasteten Knochenimplantaten ein optimaler zeitlich gesteuerter Resorbierablauf sicherstellen.

Zweckmäßig ist es, wenn das Knochenimplantat nicht hohl und nicht biegsam, wie eine endoluminale Gefäßprothese ausgebildet ist.

Die Erfindung wird nachfolgend mit Hilfe einer Zeichnung näher erläutert. Dabei werden unterschiedliche Ausführungsbeispiele in den Figuren visualisiert und nachfolgend näher erläutert.

Es zeigen:
Fig. 1 eine schematische Ansicht eines Ausgangscompositmaterials, aus der das Knochenimplantat gefertigt ist und bei der die Tragstruktur in kugeliger Form vorliegt,
Fig. 2 eine schematische Ansicht eines Ausgangscompositmaterials, aus der das Knochenimplantat gefertigt ist und bei der die Tragstruktur in faserartiger oder plattenartiger Form vorliegt,
Fig. 3 eine schematische Ansicht eines Ausgangscompositmaterials, aus der das Knochenimplantat gefertigt ist und bei der die Tragstruktur in stabartiger Form vorliegt,
Fig. 4 eine schematische Ansicht eines Ausgangscompositmaterials, aus der das Knochenimplantat gefertigt ist und bei der die Tragstruktur nach einer ersten Art von Verstrebungen vorliegt,
Fig. 5 eine schematische Ansicht eines Ausgangscompositmaterials, aus der das Knochenimplantat gefertigt ist und bei der die Tragstruktur nach neiner zweiten Art von Verstrebungen vorliegt,
Fig. 6 eine schematische Ansicht eines Ausgangscompositmaterials, aus der das Knochenimplantat gefertigt ist und bei der die Tragstruktur in bienenwabenartiger Form vorliegt,
Fig. 7 eine schematische Ansicht eines Ausgangscompositmaterials, aus der das Knochenimplantat gefertigt ist und bei der die Tragstruktur panzersperrenartiger Form vorliegt, und
Fig. 8 eine schematische Ansicht eines Ausgangscompositmaterials, aus der das Knochenimplantat gefertigt ist und bei der die Tragstruktur gitterartiger Form vorliegt.

Die Figuren sind lediglich schematischen Natur und dienen nur dem Verständnis der Erfindung.

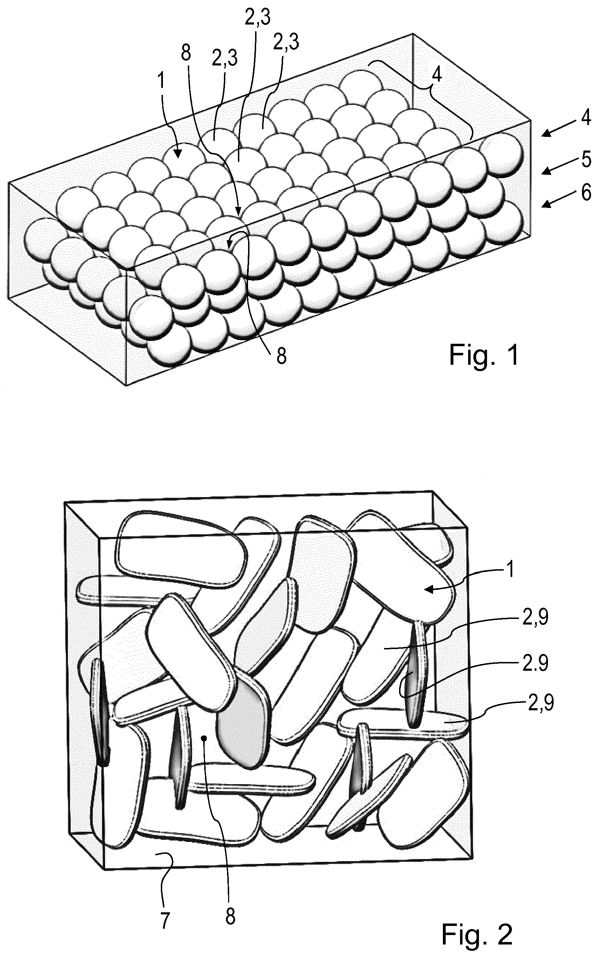

In der Fig. 1 ist ein Ausgangsmaterial für ein Knochenimplantat dargestellt, wobei die spezielle Form des Knochenimplantats nicht wiedergegeben ist. Möglich ist, dass ein solches Knochenimplantat die endgültige Form einer Platte, eines Craniumimplantats, eines Nagels, eines Gitters, eines Gewebes, eines Niets oder einer Schraube aufweist. Spanabhebende und spanlose Formverfahren sind einsetzbar, um die endgültige Form des Implantats zu erhalten.

Das Ausgangsmaterial, aus dem das erfindungsgemäße Knochenimplantat gefertigt wird, weist in der in Fig. 1 dargestellten Ausführungsform eine Tragstruktur 1 auf, das Tragelemente 2 aufweist, die eine Kugelform haben. Die Tragelemente 2 haben also die Form von Kugeln 3.

Mehrere Kugeln 3 sind miteinander in Kontakt und in einer oder mehreren Lagen, etwa einer ersten Lage 4 angeordnet. Es gibt noch weitere Kugeln 3 in weiteren Lagen. Im hier dargestellten Ausführungsbeispiel ist eine weitere, zweite Lage an Kugeln 3 und eine weitere, dritte Lage an Kugeln 3 vorhanden. Während die erste Lage mit dem Bezugszeichen 4 referenziert ist, ist die zweite Lage mit dem Bezugszeichen 5 referenziert und die dritte Lage mit dem Bezugszeichen 6 referenziert.

Eine Kugel 3 der zweiten Lage ist, außer sie ist am Rand der Lage befindlich, mit vier Kugeln 3 derselben Lage in Kontakt. Zumindest eine Kugel 3 der ersten Lage 4 und eine Kugel 3 der dritten Lage 6 berührt die besagte Kugel 3 der zweiten Lage 5 ebenso.

Im dargestellten Ausführungsbeispiel, sind jedoch die Kugeln 3 der einzelnen Lagen nach Art einer dichtesten Kugelpackung angeordnet. Es sind somit hexagonale Kugelschichten vorhanden. Neben einer solch hexagonalen dichtesten Kugelpackung ist aber auch eine kubisch dichteste Kugelpackung möglich. Auch ist der Strukturtyp "dhcp" möglich. Als Alternative ist es natürlich auch möglich, dass die einzelnen Kugeln 3, nach Art eines kubisch raumzentriertes Gitters (bcc) angeordnet sind.

Alle Kugeln 3, also alle Kugeln 3 aller unterschiedlichen Lagen an Tragelementen 2, sind von einer bio-degradierbaren und/oder bio-resorbierbaren Schutzstruktur 7 umgeben. In Hohlräumen 8, zwischen den einzelnen kugelförmigen Tragelementen 2 ist die Schutzstruktur 7 ebenfalls eingedrungen, so dass die Kugeln 3 unterschiedlich in der Schutzstruktur 7 gehalten sind.

Die Kugeln 3, welche mit anderen Kugeln 3 in Verbindung stehen, also die sich kontaktierenden Tragelemente 2, sind stoffschlüssig miteinander verbunden, bspw. verschweißt. Im vorliegenden Fall wurde auf ein Sinterverfahren zurückgegriffen, um die Tragelemente 2 miteinander zu verbinden. Als besonders wirkungsvoll hat es sich herausgestellt, wenn ein Laser auf das in Pulverform vorliegende Material der Tragelemente 2 einwirkt.

Durch die geschickte Anordnung der Tragelemente 2 aneinander, wird eine Wegverlängerung für korrosives Material geschaffen. Das korrosive Material, das durch, bspw. eine Fraktur in der Schutzstruktur 7 in das Innere gelangt, muss von einem, für den korrosiven Angriff empfänglichen Tragelement 2 zum nächsten, einen besonders langen Weg zurücklegen, um dieses weitere Tragelement 2 überhaupt angreifen zu können. Das hat Auswirkungen auf die Dauer der Korrosion. Es dauert besonders lange, bis das nächste Tragelement 2 angegriffen und abgebaut ist. Folglich dauert es besonders lange, bis die Tragstruktur 1 geschwächt ist und irgendwann alle Tragelemente 2 abgebaut sind. Auf diese Weise kann man gezielt, mittels der Anordnung der einzelnen Tragelemente 2 im Inneren der Schutzstruktur 7 die Abbaukinetik einstellen. Diese ist abhängig, von der jeweiligen Materialverwendung in der Tragstruktur 1 und der Schutzstruktur 7. Die diesbezüglich eingesetzten Werkstoffe können gezielt und aufeinander für das gewünschte Ziel abgestimmt ausgewählt werden.

Diesem Prinzip wird auch in den in den Fig. 2 bis 8 dargestellten Ausführungsbeispielen gefolgt. Auch dort sind die einzelnen Tragelemente 2 immer stoffschlüssig miteinander verbunden, vorzugsweise verschweißt bzw. sinterartig miteinander verbunden. Das fertige Knochenimplantat ist möglichst allseitig von der Schutzstruktur 7 umgeben. Grundsätzlich ist es jedoch auch möglich, dass Teile der Tragstruktur 1 an die Oberfläche des fertigen Knochenimplantats ragen.

Die in den Ausführungsbeispielen der Fig. 2 bis 8 verwendeten Tragelemente 2 der Tragstruktur 1 weisen unterschiedliche Formen auf.

So sind die Tragelemente 2 des Ausführungsbeispiels gemäß Fig. 2 als Fasern 9 bzw. als Platten ausgebildet. Die Fasern 9 sind ebene plattenartige Strukturen, die teilweise auch miteinander verschnitten sein können. Es können aber auch Filamente sein. Sie haben an ihren Rändern Abrundungen, können jedoch auch eckig ausgestaltet sein oder gar spitz zulaufen. Auch ist es möglich, dass die Fasern selber als Art Platten ausgebildet sind und dann nicht eben sind, sondern eine wellige Form, bspw. eine konvexe oder konkave Form haben. Sie können eine gleichbleibende Dicke aufweisen, können aber auch ellipsoidartig ausgebildet sein. Insbesondere ist eine Linsenform möglich.

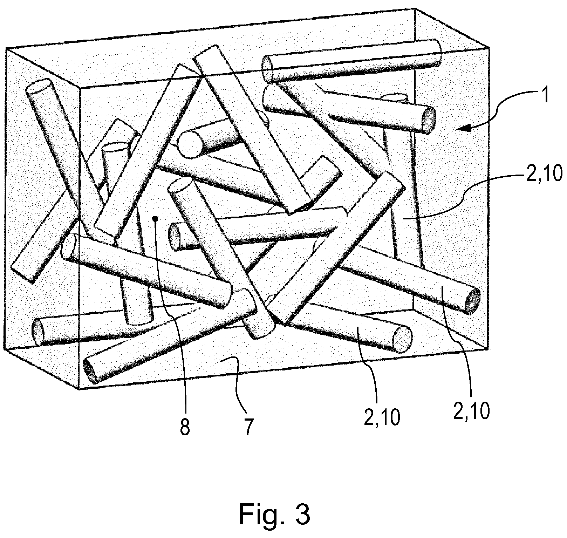

In dem Ausführungsbeispiel gemäß Fig. 3 sind die Tragelemente 2 als Stäbe ausgeformt. Die Stäbe haben dabei das Bezugszeichen 10 und sind säulenartig. Sie haben einen kreisrunden Querschnitt, können aber auch einen PolygonQuerschnitt haben. Die Stäbe 10 haben einen gleichbleibenden Querschnitt. Der Querschnitt kann sich jedoch aber auch verändern.

Wenigstens ein Stab 10 ist mit einem weiteren Stab 10 in Kontakt und im Bereich des Kontakts, wie schon bzgl. der Ausführungsbeispiele der Fig. 1 und 2 erläutert, stoffschlüssig verbunden. Es hat sich als vorteilhaft herausgestellt, wenn jeweils 2, 3, 4, 5, 6, 7, 8, 9, 10, 11 und mehr Stäbe miteinander verbunden sind. Auch hier, wie schon in den beiden vorab erläuterten Ausführungsbeispielen, durchzieht die Tragstruktur 1 die Schutzstruktur 7 bzw. wird von ihr umgeben.

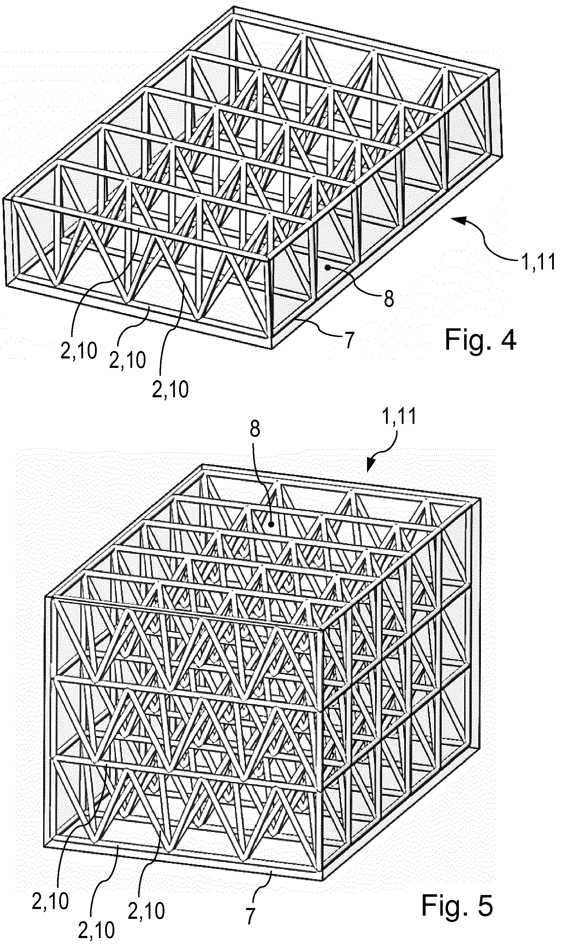

Während in dem Ausführungsbeispiel gemäß Fig. 3 die einzelnen Stäbe 10 nahezu wirr im Inneren der Schutzstruktur 7 befindlich sind, sind die Stäbe 10 in dem Ausführungsbeispiel der Fig. 4 und 5 miteinander nach Art von Verstrebungen 11 einer sich geometrisch wiederholenden Anordnung positioniert. Die Verstrebung 11 ist auch punktsymmetrisch ausbildbar.

Die Ausführungsbeispiele der Fig. 4 und 5 unterscheiden sich in der Anzahl der Lagen an Verstrebungen 11. So sind in dem Ausführungsbeispiel gemäß Fig. 5 drei Lagen an Verstrebungen 11 vorhanden, wohingegen in dem Ausführungsbeispiel gemäß Fig. 4 nur eine Lage an Verstrebungen 11 vorhanden ist.

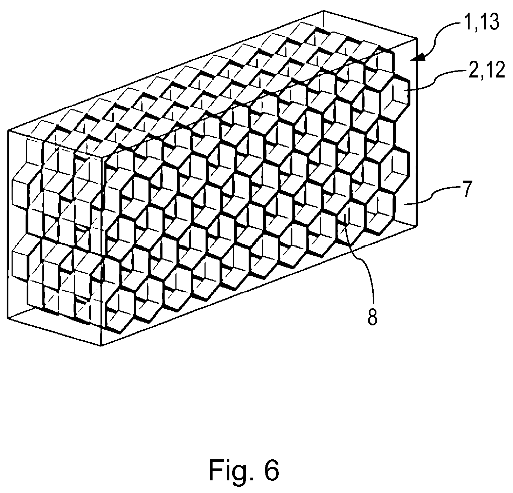

In dem Ausführungsbeispiel gemäß Fig. 6 sind die einzelnen Tragelemente 2 nach Art von Platten 12 ausgebildet, wobei die einzelnen Platten 12 Lagen einer bienenwabenähnlichen Struktur formen, wobei einzelne Lagen der bienenwabenähnlichen Struktur zueinander versetzt sind. Die einzelnen Platten 12 formen somit eine komplexe Bienenwabenstruktur 13 aus.

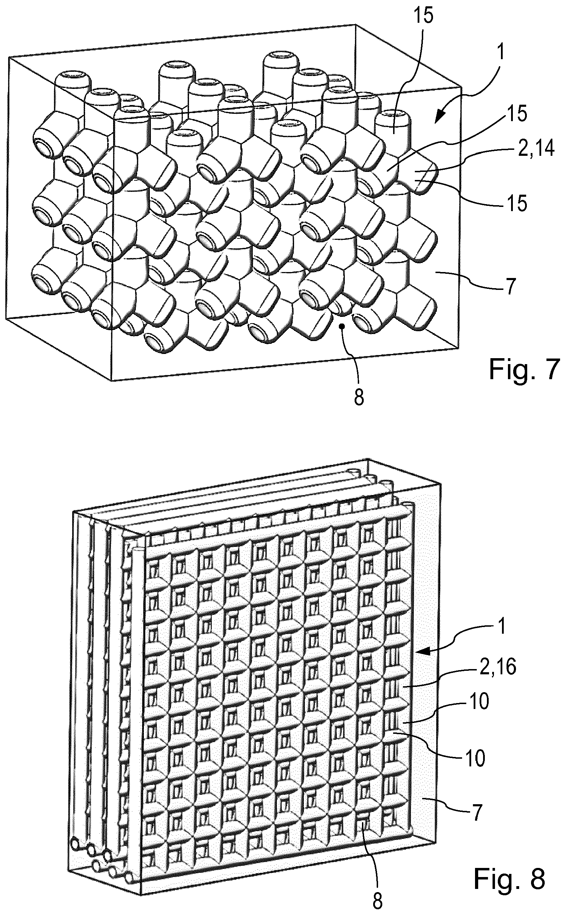

In Fig. 7 ist die Tragstruktur 1 aus einzelnen Tragelementen 2 zusammengesetzt, die nach Art von Panzersperrenelementen 14 ausgeformt sind. Dabei weist ein Panzersperrenelement 14 vier zylindrische Teilsegmente 15 auf. Jedes der vier zylindrischen Teilsegmente 15 weist zum nächstgelegenen zylindrischen Teilsegment 15 desselben Panzersperrenelementes 14 denselben Raumwinkel auf. Die zylindrischen Teilsegmente 15 sind an den Enden abgerundet, können jedoch auch unabgerundete Kanten aufweisen. Die einzelnen zylindrischen Teilsegmente 15 können aus Vollmaterial bestehen, können jedoch auch hohl sein.

Die Hohlräume 8 sind wiederum durch die Schutzstruktur 7 ausgefüllt.

Die einzelnen zylindrischen Teilsegmente 15 können einen gleichbleibenden Durchmesser haben oder einen sich verändernden Durchmesser haben. Die einzelnen zylindrischen Teilsegmente 15 können den gleichen Durchmesser bzw. den gleichen Durchmesserverlauf aufweisen, wie die benachbarten zylindrischen Teilsegmente, oder einen davon unterschiedlichen Durchmesser bzw. Durchmesserverlauf haben.

In Fig. 8 ist ein weiteres Ausführungsbeispiel einer erfindungsgemäßen Tragstruktur 1 in einem erfindungsgemäß herzustellenden Knochenimplantat dargestellt. Dabei sind die einzelnen Tragelemente 2 der Tragstruktur 1 nach Art eines Gitters 16 vorzugsweise in unterschiedlichen Lagen, miteinander kombiniert. Die einzelnen Stäbe 10 des Gitters 16 kreuzen sich orthogonal. Um eine möglichst große mechanische Festigkeit, insbesondere nach der Implantierung, zu erreichen, ist es von Vorteil, wenn die einzelnen Tragelemente möglichst dicht aneinander anliegend in der Schutzstruktur 7 vorhanden sind.

Eine Ausgestaltung ähnlich eines Einkornbetons ist grundsätzlich auch möglich.

### Bezugszeichenliste

- 1: Tragstruktur
- 2: Tragelemente
- 3: Kugel
- 4: erste Lage
- 5: zweite Lage
- 6: dritte Lage
- 7: Schutzstruktur
- 8: Hohlraum
- 9: Faser
- 10: Stab
- 11: Verstrebung
- 12: Platte
- 13: komplexe Bienenwabenstruktur
- 14: Panzersperrenelement
- 15: zylindrisches Teilsegment
- 16: Gitter

## Patentansprüche

1. Knochenimplantat mit einer Tragstruktur (1) aus einer Metalllegierung und mit einer bio-degradierbaren und resorbierbaren Schutzstruktur (7), wobei die Schutzstruktur (7) einen von der Tragstruktur (1) unterschiedlichen Werkstoff aufweist und wobei die Tragstruktur (1) von der Schutzstruktur (7) umgeben und/oder durchsetzt ist, wobei die Schutzstruktur (7) so an und/oder auf der Tragstruktur (1) angebracht ist, dass die Tragstruktur (1) vor Kontakt mit einer aggressiven Körperflüssigkeit in einer in einem Knochen eines Lebewesens, wie eines Säugetiers, verankerten Position geschützt ist, **dadurch gekennzeichnet, dass** die Tragstruktur (1) einzelne miteinander stoffschlüssig verbundene Tragelemente (2) aufweist.

2. Knochenimplantat nach Anspruch 1, **dadurch gekennzeichnet, dass** die Metalllegierung ein bio-degradierbarer und resorbierbarer Werkstoff ist.

3. Knochenimplantat nach Anspruch 1 oder 2, **dadurch gekennzeichnet, dass** der Werkstoff der Tragstruktur (1) zumindest ein Element oder mehrere Elemente der Gruppe bestehend aus Magnesium, Eisen, Zink, Strontium, Fluor, Mangan und Calcium sowie deren mögliche Ionen aufweist.

4. Knochenimplantat nach einem der Ansprüche 1 bis 3, **dadurch gekennzeichnet, dass** die Schutzstruktur (7) aus dem von der Tragstruktur (1) unterschiedlichen Werkstoff besteht.

5. Knochenimplantat nach einem der Ansprüche 1 bis 4, **dadurch gekennzeichnet, dass** die Schutzstruktur (7) nicht-metallisch ist, vorzugsweise aus Kunststoff besteht, und weiter vorzugsweise Polylactid-Verbindungen aufweist.

6. Knochenimplantat nach einem der Ansprüche 1 bis 5, **dadurch gekennzeichnet, dass** die Schutzstruktur (7) Polymilchsäure, wie PLA, und/ oder Polyglykolsäure (PGA) und/oder Poly-Caprolacton (PCL), wie Poly-ε-Caprolacton-co-Lactid und/oder PDA, PDLLA-TCP und/oder PDLLA-Kalziumcarbonat aufweist.

7. Knochenimplantat nach einem der Ansprüche 1 bis 6, **dadurch gekennzeichnet, dass** die Tragstruktur (1) und die Schutzstruktur (7) nach Art eines Material-Composit-Bauteils oder eines Hybridbauteils voneinander durchdrungen oder miteinander vermischt sind.

8. Knochenimplantat nach einem der Ansprüche 1 bis 7, **dadurch gekennzeichnet, dass** die Tragstruktur (1) eine Form aneinandergesetzter Tragpartikel, wie Körnern, und/oder Kugeln (3) und/oder Fachwerkelementen, wie Stäben (10), und/oder Hexagonalelementen und/oder Dreiecken und/oder krähenfußartiger Elementen und/oder Waben und/oder Fasern (9) aufweist.

9. Knochenimplantat nach einem der Ansprüche 1 bis 7, **dadurch gekennzeichnet, dass** die Tragstruktur (1) die Struktur eines Metallschaumes aufweist.

10. Knochenimplantat nach einem der Ansprüche 8 oder 9, **dadurch gekennzeichnet, dass** Tragpartikel der Tragstruktur (1) so aneinander grenzen, dass zwischen ihnen Hohlräume befindlich sind.

11. Knochenimplantat nach Anspruch 10, **dadurch gekennzeichnet, dass** zumindest ein Hohlraum oder vorzugsweise eine Vielzahl von Hohlräumen vorzugsweise alle Hohlräume, mit dem Material der Schutzstruktur (7) zumindest teilweise, vorzugsweise vollständig befüllt sind.

12. Knochenimplantat nach Anspruch 11, **dadurch gekennzeichnet, dass** einige Hohlräume vollständig mit Material der Schutzstruktur (7) ausgefüllt sind und einige Hohlräume nur teilweise mit Material der Schutzstruktur (7) ausgefüllt sind, und vorzugsweise das Verhältnis von vollständig ausgefüllten Hohlräumen zu teilweise ausgefüllten Hohlräumen 20:1 bis 10:1, weiter vorzugsweise das Verhältnis ungefähr 15:1 ist.

13. Knochenimplantat nach einem der Ansprüche 1 bis 12, **dadurch gekennzeichnet, dass** die Tragstruktur (1) mit der Schutzstruktur (7) verschränkt und/oder in Sandwich-Bauart miteinander kombiniert ist.

14. Knochenimplantat nach einem der Ansprüche 1 bis 13, **dadurch gekennzeichnet, dass** die Tragstruktur (1) eine höhere bspw. mechanische Festigkeit als die Schutzstruktur (7) aufweist.

15. Knochenimplantat nach einem der Ansprüche 1 bis 14, **dadurch gekennzeichnet, dass** das Knochenimplantat die Form einer Platte, eines Craniumimplantats, eines Nagels, eines Gitters, eines Gewebes, eines Niets oder einer Schraube aufweist.

## Claims

1. A bone implant comprising a support structure (1) made of a metal alloy and comprising a biodegradable and absorbable protective structure (7), wherein the protective structure (7) includes a material different from the support structure (1) and the support structure (1) is surrounded by and/or interspersed with the protective structure (7), wherein the protective structure (7) is arranged at and/or on the support structure (1) so that the support structure (1) is protected from contacting any aggressive body fluid in a position anchored in a bone of an individual such as a mammal, **characterized in that** the support structure (1) comprises a plurality of individual support elements (2) that are materially bonded with each other.

2. The bone implant according to claim 1, **characterized in that** the metal alloy is a biodegradable and absorbable material.

3. The bone implant according to claim 1 or 2, **characterized in that** the material of the support structure (1) includes at least one element or plural elements of the group consisting of magnesium, iron, zinc, strontium, fluorine, manganese and calcium as well as the possible ions thereof.

4. The bone implant according to any one of the claims 1 to 3, **characterized in that** the protective structure (7) is made of a material different from the support structure (1).

5. The bone implant according to any one of the claims 1 to 4, **characterized in that** the protective structure (7) is non-metallic, preferably is made of plastic material and further preferably includes polylactide compounds.

6. The bone implant according to any one of the claims 1 to 5, **characterized in that** the protective structure (7) includes polylactic acid such as PLA and/or polyglycollic acid (PGA) and/or poly-caprolactone (PCL) such as poly-ε-caprolactone-co-lactide and/or PDA, PDLLA-TCP and/or PDLLA calcium carbonate.

7. The bone implant according to any one of the claims 1 to 6, **characterized in that** the support structure (1) and the protective structure (7) are penetrated by or mixed with each other in the way of a material composite component or a hybrid component.

8. The bone implant according to any one of the claims 1 to 7, **characterized in that** the support structure (1) exhibits a form of adjacent supporting particles such as grains and/or balls (3) and/or lattice elements such as bars (10) and/or hexagonal elements and/or triangles and/or crow's foot elements and/or honeycombs and/or fibers (9).

9. The bone implant according to any one of the claims 1 to 7, **characterized in that** the support structure (1) has the structure of metal foam.

10. The bone implant according to any one of the claims 8 or 9, **characterized in that** supporting particles of the support structure (1) are adjacent to one another so that cavities are provided there between.

11. The bone implant according to claim 10, **characterized in that** at least one cavity or preferably a plurality of cavities, preferably all cavities, are filled at least partially, preferably completely with the material of the protective structure (7).

12. The bone implant according to claim 11, **characterized in that** some cavities are filled completely with material of the protective structure (7) and some cavities are filled only partially with material of the protective structure (7), and preferably the ratio of completely filled cavities to partially filled cavities is 20:1 to 10:1, further preferably the ratio is approximately 15:1.

13. The bone implant according to any one of the claims 1 to 12, **characterized in that** the support structure (1) is entangled and/or combined in sandwich construction with the protective structure (7).

14. The bone implant according to any one of the claims 1 to 13, **characterized in that** the support structure (1) exhibits higher, e.g., mechanical strength than the protective structure (7).

15. The bone implant according to any one of the claims 1 to 14, **characterized in that** the bone implant is in the form of a plate, a cranium implant, a nail, a lattice, a fabric, a rivet or a screw.

## Revendications

1. Implant osseux avec une structure de support (1) en un alliage métallique et avec une structure de protection biodégradable et absorbable (7), dans lequel la structure de protection (7) présente un matériau différent de la structure de support (1) et dans lequel la structure de support (1) est entourée et/ou traversée par la structure de protection (7), dans lequel la structure de protection (7) est agencée de telle manière dans et/ou sur la structure de support (1) que la structure de support (1) soit protégée contre un contact avec un liquide corporel agressif dans une position ancrée dans un os d'un être vivant, tel qu'un mammifère, **caractérisé en ce que** la structure de support (1) présente des éléments de support individuels (2) reliés matériellement les uns aux autres.

2. Implant osseux selon la revendication 1, **caractérisé en ce que** l'alliage métallique est un matériau biodégradable et absorbable.

3. Implant osseux selon la revendication 1 ou 2, **caractérisé en ce que** le matériau de la structure de support (1) présente au moins un élément ou plusieurs éléments du groupe composé du magnésium, du fer, du zinc, du strontium, du fluor, du manganèse et du calcium ainsi que de leurs ions possibles.

4. Implant osseux selon l'une quelconque des revendications 1 à 3, **caractérisé en ce que** la structure de protection (7) se compose du matériau différent de la structure de support (1).

5. Implant osseux selon l'une quelconque des revendications 1 à 4, **caractérisé en ce que** la structure de protection (7) n'est pas métallique, se compose de préférence de matière synthétique et présente de préférence encore des composés polylactides.

6. Implant osseux selon l'une quelconque des revendications 1 à 5, **caractérisé en ce que** la structure de protection (7) présente de l'acide polylactique, comme PLA, et/ou de l'acide polyglycolique (PGA) et/ou du poly-caprolactone (PCL), comme le poly-ε-caprolactone-co-lactide et/ou du PDA, du PDLLA-TCP et/ou du PDLLA-carbonate de calcium.

7. Implant osseux selon l'une quelconque des revendications 1 à 6, **caractérisé en ce que** la structure de support (1) et la structure de protection (7) sont pénétrées l'une dans l'autre ou mélangées l'une avec l'autre à la manière d'un élément en matériau composite ou d'un composant hybride.

8. Implant osseux selon l'une quelconque des revendications 1 à 7, **caractérisé en ce que** la structure de support (1) présente une forme de particules de support juxtaposées, comme des grains et/ou des sphères (3) et/ou des éléments de treillis, comme des barres (10), et/ou des éléments hexagonaux et/ou des triangles et/ou des éléments en forme de pattes d'oie et/ou des nids d'abeilles et/ou des fibres (9).

9. Implant osseux selon l'une quelconque des revendications 1 à 7, **caractérisé en ce que** la structure de support (1) présente la structure d'une mousse métallique.

10. Implant osseux selon l'une des revendications 8 ou 9, **caractérisé en ce que** des particules de support de la structure de support (1) sont jointives l'une à l'autre de telle manière qu'il se trouve des espaces vides entre elles.

11. Implant osseux selon la revendication 10, **caractérisé en ce qu'**au moins un espace vide ou de préférence une multiplicité d'espaces vides de préférence tous les espaces vides sont remplis au moins partiellement, de préférence entièrement avec le matériau de la structure de protection (7).

12. Implant osseux selon la revendication 11, **caractérisé en ce que** certains espaces vides sont remplis entièrement avec du matériau de la structure de protection (7) et certains espaces vides ne sont remplis que partiellement avec du matériau de la structure de protection (7), et de préférence le rapport des espaces vides remplis entièrement aux espaces vides remplis partiellement est de 20:1 à 10:1, et de préférence encore le rapport vaut environ 15:1.

13. Implant osseux selon l'une quelconque des revendications 1 à 12, **caractérisé en ce que** la structure de support (1) est entrelacée avec la structure de protection (7) et/ou elles sont combinées l'une à l'autre à la manière d'un sandwich.

14. Implant osseux selon l'une quelconque des revendications 1 à 13, **caractérisé en ce que** la structure de support (1) présente une plus grande résistance, par exemple mécanique, que la structure de protection (7).

15. Implant osseux selon l'une quelconque des revendications 1 à 14, **caractérisé en ce que** l'implant osseux présente la forme d'une plaque, d'un implant crânien, d'un clou, d'une grille, d'un tissu, d'un rivet ou d'une vis.
